# EUROPEAN PATENT SPECIFICATION

(11) **EP 4 590 889 B1**
(45) Date of publication and mention of the grant of the patent: **24.09.2025**
(21) Application number: 24761640.2
(22) Date of filing: 27.08.2024
(51) Int. Cl.: D04H 3/007, D04H 3/018, D04H 3/147, A61F 13/49, B32B 5/02, B32B 5/26

(54) **HIGH CROSS-DIRECTIONAL ELONGATION NONWOVEN FABRICS**
VLIESSTOFFE MIT HOHER QUERDEHNUNG
TISSUS NON TISSES A ALLONGEMENT TRANSVERSAL ELEVE

(30) Priority: 28.08.2023 EP 23193634
(43) Date of publication of application: 30.07.2025
(73) Proprietor: Fibertex Personal Care A/S, 9220 Aalborg (DK)
(72) Inventor: TAIDAL, Lasse Kristoffer, 9000 Aalborg (DK); UDENGAARD, Brian, 8520 Lystrup (DK)
(74) Representative: Frick, Robert
(86) International application number: PCT/EP2024/073884
(87) International publication number: WO 2025/045858

(56) References cited:
- EP-A1- 3 246 444
- EP-A1- 4 180 017
- WO-A1-2017/198336
- WO-A1-2023/083600

## Description

The invention relates to stretchable nonwoven sheets comprising two spunbonded facing layers and a meltblown middle layer, and a method of making these sheets.

The hygiene industry uses nonwoven sheets on a large scale as materials in baby diapers and adult incontinence products. Exemplary nonwoven products for use in baby diapers and adult incontinence products, and methods for making them, are disclosed in EP 3 246 444 A1, WO 2017/198336 A1, WO 2023/083600 A1 and EP 4 180 017 A1.

In many instances, for example to make back-ears in open diapers, the materials are required to be elastically stretchable. One option to make elastically stretchable materials is laminating stretchable nonwovens and elastic films or strands. The elastic film imparts the elastic properties, while the nonwoven sheets layers make the material look and feel as an integrated part of the diaper. In such configuration, while the nonwoven sheets do not need to have elastic properties themselves, their elongation, meaning their ability to stretch, is the limiting factor for overall elongation of the back ear. Hence, the nonwoven material should have significant elongation properties. More specifically, as in production methods of open diapers, the length direction of the back ears requiring elastic stretch is usually the cross-machine direction (CD) of the nonwoven sheets, for nonwoven sheets to be laminated with an elastic film or strand for making diaper back ears, it is advantageous to exhibit significant elongation specifically in cross-machine direction.

In the state of the art, nonwoven sheets of satisfactory inherent CD direction elongation that can be produced at reasonable cost are not available. As a workaround, it is known to pre-stretch the elastic film and apply the nonwoven sheet to both sides of the elastic laminate while it is stretched. When the elastic film is allowed to contract, the nonwoven sheets contract and drape. Downsides of this approach are an overuse of nonwovens and a higher thickness and poor visual impression of the final product. Another known method is to pre-stretch the nonwoven sheets by ring-rolling or similar methods, which leads to partly breaking the nonwoven structure and enhanced elongation properties. However, the activation leads to an additional step in production and the partly broken structure of the nonwoven creates a less desired visual impression.

An object of this invention is to provide a nonwoven sheet that has satisfactory inherent CD elongation and can be produced at reasonable cost.

Against this background, the present invention proposes a method according to claim 1 for making a nonwoven sheet, the nonwoven sheet comprising two facing layers of spunbonded nonwoven material and a middle layer of meltblown nonwoven material, wherein the spunbonded material of the facing layers comprises crimped fibers, the method comprising the following in-line steps: spinning crimped multicomponent fibers and laying them onto a moving spinbelt to form a web preceding one of the facing layers; meltblowing fibers and laying them onto the surface of the web formed in the previous step to form a web preceding the middle layer; spinning crimped multicomponent fibers and laying them onto the surface of the web formed in the previous step to form a web preceding the other one of the facing layers; and bonding the adjacent webs to form the nonwoven sheet by point-bond hot calendering. According to the invention, the fibers of the meltblown material of the middle layer comprise a thermoplastic elastomer and the temperature of the engraved roll of the calender is at least 10°C and/or 5% lower than the melting temperature of the lowest melting component of the multicomponent fibers.

The temperature of the calender rolls refers to the surface temperature of the calender rolls. In practice, the temperature is often not measured on the surface, but set by setting the temperature of a heating oil used to heat the rolls, usually by being passed through conduits inside the rolls. The relation between the oil temperature and the actual surface temperature is not linear and influenced by multiple factors. In any case, however, the surface temperature is never higher than the oil temperature. In other words, the definition that the (surface) temperature of the engraved roll of the calender is at least 10°C and/or 5% lower than the melting temperature of the lowest melting component of the multicomponent fibers encompasses a (more narrow) definition the temperature of a heating oil used to heat the rolls is at least 10°C and/or 5% lower than the melting temperature of the lowest melting component of the multicomponent fibers.

The present invention further proposes a nonwoven sheet according to claim 11, made by a method of the invention, the nonwoven sheet comprising two facing layers of spunbonded nonwoven material and a middle layer of meltblown nonwoven material, wherein the spunbonded material of the facing layers comprises crimped fibers, wherein the meltblown material of the middle layer comprises a thermoplastic elastomer, and wherein the nonwoven sheet is underbonded.

Point-bond hot calendering involves the use of a two-roll nip consisting, in most cases, of a heated engraved roll (e-roll) and a typically also heated smooth roll (s-roll). The multilayer web is fed by an apron leading to a calender nip and the fiber temperature is locally raised to the point at which tackiness and melting cause fiber segments caught between the tips of engraved points and the smooth roll to adhere together.

The process temperature and, in particular, the temperature of the engraved calender roll is a significant parameter for bonding quality. For a given nip line pressure, e.g. 20 to 100 N/mm, preferably 30 to 70 N/mm, and line speed, e.g. 100 to 400 m/min, preferably 200 to 350 m/min, the breaking strength reaches a maximum at a critical bonding temperature. When temperature of the engraved roll of the calender is significantly lower than the melting temperature of the lowest melting component of the multicomponent fibers, it will be significantly below the critical bonding temperature, and the sheet, specifically the spunbonded nonwoven material will become underbonded. This means that the quantity of adhered fiber segments at the engraved points is significantly reduced when compared to a critical bonding temperature.

In the nonwoven sheet made according to the invention, the meltblown middle layer acts as an adhesive to the spunbonded facing layers. Spunbonded materials comprising crimped fibers have a certain inherent elongation. The inherent elongation of even these materials, however, is still limited. Inherent elongation can be increased by underbonding. However, when underbonding sheets merely consisting of one or more layers of spunbonded material, the sheets tend to be unstable and fuzzy. The present invention uses a meltblown layer of thermoplastic elastomer material between two facing layers of spunbonded material. The meltblown layer acts as an adhesive between the facing layers due to an inherent stickiness of the freshly formed thermoplastic elastomer meltblown fibers. It was found that the stickiness of the elastic meltblown layer enables for underbonding the facing layers without detriment to fabric stability. At the same time, as the meltblown fibers are made from a thermoplastic elastomer and the layer is hence stretchable in itself, the additional layer does not hinder elongation, at least to an extent that would cancel out the positive effects of the ability to underbond.

In contrast to technologies using spunbonded layers formed from thermoplastic elastomer materials, with an aim to provide nonwoven materials that are inherently elastically stretchable and do not need to be laminated with elastic films or strands, the sheets of the present invention are very stretchable, but have no or only little inherent elasticity. In other words, when stretching the sheets of the invention, at least part of the deformation will be permanent. The meltblown layer, while formed from a thermoplastic elastomer, only functions as a connecting element for structural integrity of an underbonded web, but does not have sufficient strength to impart inherent elastic properties to the sheet.

If reference is made herein to the temperature of the engraved roll of the calender being at least a certain percentage, e.g. 5% lower than the melting temperature of the lowest melting component of the multicomponent fibers, reference is made to the melting temperature as expressed in °C. Hence, to give an example, for a polymer component of a 140°C melting point, 5% would be 7°C, and at least 5% lower than 140°C, in absolute numbers, would be 133°C or lower.

In embodiments, the temperature of the engraved roll of the calender can at least 20°C, or at least 30°C, or at least 40°C lower than the melting temperature of the lowest melting component of the multicomponent fibers. In embodiments, the temperature of the engraved roll of the calender is least 10%, or at least 20%, or at least 30% lower than the melting temperature of the lowest melting component of the crimped multicomponent fibers. In embodiments, the absolute temperature of the engraved roll of the calender can be 130°C or less, 120°C or less, 110°C or less, 110°C or less, or even 90°C or less. As explained further above, the temperature refers to the surface temperature of the roll in principle, but the feature encompasses a (more narrow) definition the temperature of a heating oil used to heat the rolls is at least a certain absolute temperature (°C) or relative temperature (%) lower than the melting temperature of the lowest melting component of the multicomponent fibers, or is a certain temperature (°C) or less.

In embodiments, the temperature of the smooth roll of the calender is smaller than the temperature of the engraved roll of the calender. Preferably, the temperature difference between the rolls is less than 20°C. Also here, the definition, in embodiments, can be applicable to both a comparison of the surface temperatures of the rolls and the temperature of heating oil used to heat the rolls.

The fibers forming for the facing layers can comprise crimped multicomponent fibers or consist of crimped multicomponent fibers. For sufficient elongation, it is preferred that at least 70 wt.%, preferably at least 80 wt.%, more preferably at least 90 wt.% of the fibers are crimped multicomponent fibers. The crimped multicomponent fibers make these layers high loft with improved softness, in addition to an improved flexibility and elongation.

The crimped multicomponent fibers are of any asymmetric cross-sectional distribution and are preferably bicomponent fibers. Preferred are side-by-side fibers, but they can also be of eccentric-sheath-core or other known configuration. In a preferred embodiment, one of the components of the fibers is polypropylene (PP) and another component of the fibers is a propylene-α-olefin copolymer material (co-PP). The copolymer is preferably a poly(propylene-ethylene) copolymer. In another embodiment, the components are both polypropylene, with the polypropylene differing from each other in properties such that a crimp in the fibers results.

The melting temperatures of polypropylene (PP) materials as used in the facing layers can, in one embodiment, range from 150-170°C, preferably 155-165°C. The melting temperatures of propylene-α-olefin copolymer material (co-PP) materials as used in the facing layers can, in one embodiment, range from 140-160°C, preferably 145-155°C. Melting temperatures (Tₘ) as given herein are generally to be understood as having been determined with DSC according to ISO 11357-3.

Typical values for melt flow rates of the polymers used in the bicomponent fibers can be between 10 and 50 g/10min. Melt flow rates as indicated herein are generally to be understood as being determined according to ISO 1133 with conditions being 230°C and 2.16 kg.

The weight ratio of the one component to the other component preferably lies between 20/80 and 80/20, more preferably between 30/70 and 70/30, and yet more preferably between 40/60 and 60/40.

Each spunbonded facing layer can comprise one single layer or can comprise two or even more layers in an SS configuration.

For sufficient stickiness, it is preferred that at least 10 wt.%, preferably at least 50 wt.%, more preferably at least 90 wt.% of the material forming for the meltblown fibers of the middle layer is a thermoplastic elastomer. In an embodiment, the meltblown fibers consist of 100 wt.% thermoplastic elastomer. From a viewpoint of stickiness, 100 wt.% of TPE can be preferred. For processability, however, mixing with another thermoplastic polymer like a regular thermoplastic polypropylene can be of advantage. When another thermoplastic polymer is used in conjunction with the TPE material for making the meltblown fibers, it is preferred that this material has a melt flow rate. The melt flow rates of the polymer(s) used for meltblowing are typically higher than that of the polymers used for spunbonding. Typical values exceed 500 g/10min determined according to ISO 1133 with conditions being 230°C and 2.16 kg.

The thermoplastic elastomer can be a thermoplastic polyolefin elastomer (TPE-o), preferably a thermoplastic polyolefin elastomer comprising propylene-α-olefin copolymers. Suitable TPE-o materials for use in the context of the present invention are comprised, for example, in the Vistamaxx^{™} series from ExxonMobil. An example comprises Vistamaxx^{™} EXP520. Alternatively or additionally, meaning as a mixture, other thermoplastic elastomer materials like especially thermoplastic polyurethanes (TPU) or styrenic block copolymers (TPE-s) may be used. In one embodiment, up to 20 wt.-% and preferably up to 10 wt.-% of a thermoplastic olefin, such as a homopolypropylene may be contained in the thermoplastic elastomer material next to the thermoplastic elastomer.

The meltblown middle layer can comprise one single layer or can comprise two or even more layers in an MM configuration.

The nonwoven sheet according to the invention, in cross-machine direction (CD), has a tensile strength @100% elongation of less than 0,65 and preferably less than 0,55 of its tensile strength @break, with tensile strength @100% elongation and tensile strength @break both being determined according to WSP 110.4. Preferred quotients of CD tensile @100% to tensile @break are less than 0,50, less than 0,45 or even less than 0,40.

In embodiments, the nonwoven sheet according to the invention has an extension at break in cross-machine direction (CD) of at least 150%, when measured according to WSP 110.4. CD elongation at 5N values are preferably at least 75%, when measured according to WSP 110.4.

In one embodiment, the basis weight of each facing layer is 5-40 g/m², preferably between 10-25 g/m². The basis weight of the meltblown middle layer may, in one embodiment, be 10 g/m² or less.

The number of bonding points per cm² of fabric surface may be lower than 100 and preferably lower than 70, and on the other hand preferably higher than 30. The total area of the fabric surface taken up by the areal bonding points in one embodiment is less than 18 % and preferably less than 15 %, and on the other hand preferably higher than 8%.

The facing layers on either side of the middle layer may be identical or different. They can individually be configured according to embodiments as described above.

The invention further relates to a laminate comprising a nonwoven sheet according to the invention and an elastically stretchable material. Elastically stretchable materials can be elastic films or strands, elastomeric coatings, elastomeric nonwovens, scrims, or the like. Preferably, the elastically stretchable material is sandwiched between two nonwoven sheets according to the invention. Lamination can be done by ultrasonic bonding, thermal bonding, gluing, and other known techniques. When laminating, the film can in one embodiment be pre-stretched and then relaxed, so the nonwoven sheets contract and drape. While this may be considered a less than optimal solution, as compared to the prior art that uses such workaround, the higher inherent elongation of the nonwoven sheets of the invention allow for reduction in the degree of pre-stretching and hence thickness increase and material use. Also, while this may be considered a less than optimal solution, the nonwoven sheets of the invention before lamination or the laminates of the invention can be subjected to ring-rolling for pre-stretch, however, with less degree of pre-stretch and hence impact than with less stretchable nonwoven sheets of the prior art.

As an alternative, the nonwoven sheet according to the invention can also be used as a standalone product in a hygiene article where stretch-ability is needed.

Yet further, the invention relates to a hygiene article comprising a nonwoven sheet or laminate according to the invention. For example, the hygiene article can be an open diaper having diaper ears, and the nonwoven sheet or laminate according to the invention can form at least part of the diaper ears.

Further details and advantages of the invention will become apparent from the figures and examples described in the following. The figures show:
- Fig. 1:: a schematic cross-section of a nonwoven sheet of the invention; and
- Fig. 2:: an exemplary process line for manufacturing nonwoven sheets of the invention.

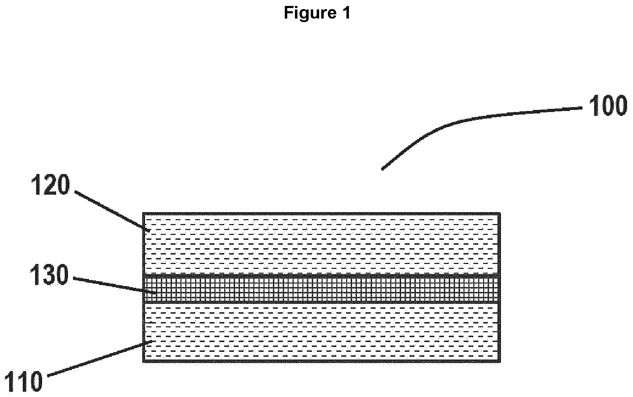

In Fig. 1 a schematic cross-section of a sandwich-type stretchable nonwoven sheet is shown. The sheet, generally designated with reference numeral 100, comprises a middle layer 130 that is covered with a facing layer 110 and 120, respectively, on either side. Both facing layers 110 and 120 are spunbonded nonwoven fabrics formed from crimped multicomponent fibers. The middle layer 130 is a meltblown nonwoven fabric formed from fibers comprising thermoplastic elastomer material. The layers 110, 120 and 130 are hot calender point-bonded to each other, but the fabric is underbonded due to using lower calender temperatures than temperatures that would usually be considered appropriate for bonding.

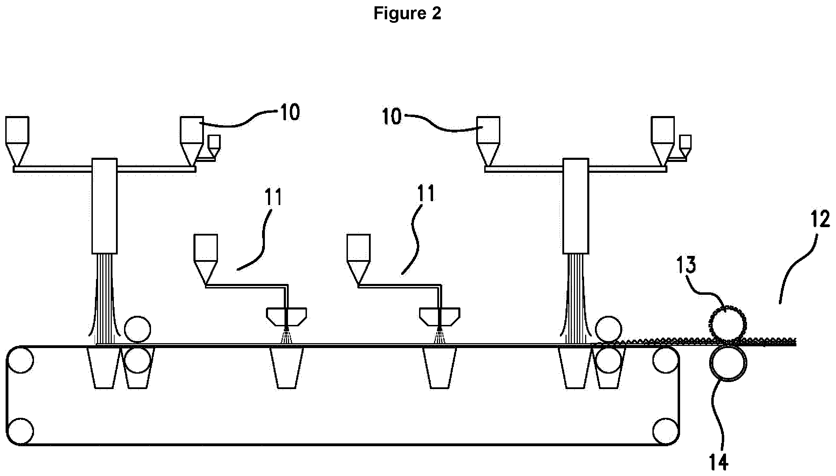

An exemplary process line for carrying out a method of the invention and manufacturing a nonwoven fabric sheet 100 as illustrated in Fig. 1 is shown in Fig. 2.

The process line comprises a conveyor belt that runs along a first spunbonding machine 10 for forming one of the facing layers 110, two meltblowing machines 11 for forming the middle layer 130, spunbonding machine 10 for forming the other of the facing layer 120, a pair of pre-compaction rolls behind every spunbonding machine 10, and downstream of the four machines 10, 11 a bonding station 12 comprising a pair of calender rolls, namely e-roll 13 and s-roll 14.

The spunbonding machines 10 each comprise two reservoirs for the polymer raw materials that form for the two components of the bicomponent fiber, a mixing and feeding channel, an extrusion die, an air channel for quenching and stretching and an air suctioning device below the conveyor belt. The meltblowing machines 11 each comprise a reservoir for the thermoplastic elastomer material, a feeder, an air manifold, a die and an air suctioning device below the conveyor belt. Pre-consolidation leads to a light fiber to fiber integration necessary to withstand further processing.

### Examples 1-7:

The examples as described in Table 1 were prepared on an SMMS (spunbonded, meltblown, meltblown, spunbonded) line as shown in Fig. 2, with the spunbonded layers being loft (L, comprising bicomponent fibers). Line parameters were a line speed of 268 m/min and a nip pressure of 52 N/mm. The bond pattern was an open dot bond pattern with a bonding area of 13.6%.

**Table 1**

| Ex. | PP S1 | coPP S1 | PP/coPP ratio S1 | BW S1 (g/m²) | PP S2 | coPP S2 | PP/coPP ratio S2 | BW S2 (g/m²) |
|---|---|---|---|---|---|---|---|---|
| 1 | 511A | 261S | 40/60 | 9,6 | 511A | 261S | 40/60 | 8,6 |
| 2 | 511A | 261S | 40/60 | 9,6 | 511A | 261S | 40/60 | 8,6 |
| 3 | 511A | 261S | 50/50 | 10,9 | 511A | 261S | 50/50 | 8,6 |
| 4 | 511A | 261S | 50/50 | 10,9 | 511A | 261S | 50/50 | 8,6 |
| 5 | 511A | 261S | 40/60 | 9,6 | 511A | 261S | 40/60 | 8,6 |
| 6 | 511A | 261S | 40/60 | 9,6 | 511A | 261S | 40/60 | 8,6 |
| 7 | 511A | 261S | 50/50 | 10,9 | 511A | 261S | 50/50 | 8,6 |

**Table 1 (cntd.)**

| Ex. | oTPE in MM | rem. MM | wt.% oTPE in MM | BW MM (g/m²) | Calender Temp. E-roll (°C) | Calender Temp. S-roll (°C) |
|---|---|---|---|---|---|---|
| 1 | | MFI800 | 0 | 3,6 | 101 | 101 |
| 2 | EXP520 | MFI800 | 75 | 3,6 | 101 | 101 |
| 3 | EXP520 | | 100 | 3,4 | 101 | 101 |
| 4 | EXP520 | | 100 | 3,4 | 130 | 120 |
| 5 | | MFI800 | 0 | 3,6 | 151 | 149 |
| 6 | EXP520 | MFI800 | 50 | 3,6 | 101 | 101 |
| 7 | EXP520 | | 100 | 3,4 | 140 | 130 |

The calender temperatures of the E- and S-rolls refer to the temperature of the oil heating the calender. The actual surface temperature of the rolls will be a bit smaller even.

The 511A polymer is a polypropylene homopolymer from the company Sabic with a narrow polymer weight distribution (M_{w}/Mₙ is 3,8), a MFR of 25 g/10min and a Tₘ of 161°C.

The 261S polymer is a polypropylene-ethylene random polymer from the company LyondellBasell with a narrow polymer weight distribution (M_{w}/Mₙ is 3,8), a MFR of 30 g/10min and a Tₘ of 153°C.

The MFI800 material is a standard polypropylene homopolymer meltblown grade with an MFR of 800 g/10min.

Vistamaxx^{™} EXP520 is a TPE-o material from Exxon mobile of suitable MFR.

Examples 1, 5 and 7 are comparative examples outside the scope of the present invention, as they are either not underbonded or do not comprise a TPE material in the meltblown core layer. Examples 2, 3, 4 and 6 are inventive examples.

As already outlined further above, the aim for the materials of the invention is to achieve CD-elongations of minimum 150% at break. In addition, it is desired to achieve easy elongation. This can be measured by the properties of tensile elongation at 5N, tensile force at 100% elongation and tensile force at 150% elongation. In addition, the slope of the tensile-elongation-graph should be taken into account to make sure that the nonwoven web is continuously intact through the stretch of the nonwoven.

Measurement results for the nonwoven sheets of Examples 1-7 are shown in Table 2.

**Table 2**

| Ex. | CD elong. at break (%) | CD elong. at 5N (%) | CD tensile at 100% elong. (N/50 mm) | CD tensile at 150% elong. (N/50 mm) | Sum of slope 50-100 % | Sum of slope 100-150 % |
|---|---|---|---|---|---|---|
| 1 | 140,2 | 127,6 | 4,0 | 4,0 | 1,5 | -0,0 |
| 2 | 171,5 | 83,8 | 5,9 | 7,1 | 4,7 | 2,5 |
| 3 | 160,3 | 70,9 | 6,5 | 7,6 | 4,9 | 2,3 |
| 4 | 153,2 | 59,9 | 7,5 | 8,8 | 6,2 | 2,6 |
| 5 | 149,3 | 74,5 | 6,2 | 7,1 | 4,3 | 1,7 |
| 6 | 176 | 93,5 | 5,4 | 6,0 | 3,6 | 1,3 |
| 7 | 157,8 | 58,0 | 7,9 | 10,0 | 6,9 | 4,3 |

All values from Table 2 were determined following the WSP 100.4 test method.

The comparison of Examples 1 and 5 (both outside the invention) demonstrates that the CD elongation at 5N becomes higher (improves) when the sheet is underbonded as in Example 1. At the same time, CD elongation at break does not achieve the desired 150% in either case and is even slightly worse in Example 1. Also, the fact that the sum of slope 100-150% for Example 1 is negative proves that the sheet of Example 1 is no longer intact in elongation ranges exceeding 100%.

In contrast, the inventive examples achieve easy elongation of the nonwoven sheet and at the same time obtain high CD-elongations at max force more than 150% while keeping the nonwoven sheet material. Therefore, the meltblown layers formed from the thermoplastic elastomer functions as an adhesive keeping the nonwoven sheet intact while making it possible to underbond the spunbonded fibers without the nonwoven sheet delaminating during the elongation.

For example, the comparison of inventive Examples 2/6, instead of comparative Example 1, with comparative Example 5 demonstrates that the underbonded sheets have different and improved properties when the thermoplastic elastomer was used for the M layer. Not only does CD elongation at break become higher and exceed the desired 150% limit, also CD elongation at 5N becomes higher (improves) and the slope 100-150% remains positive.

The fact that both the slope 100-150% as well as the absolute tensile at 150% elongation of Example 2 is higher than that of Example 6 can be regarded as an indicator that the higher content of Vistamaxx (75 wt.%) in Example 2 keeps the nonwoven material intact better than in Example 6 (50 wt.% Vistamaxx).

### Examples 8-11:

The examples as described in Table 3 were prepared on an SMMS (spunbonded, meltblown, meltblown, spunbonded) line as shown in Fig 2, with the spunbonded layers being loft (L, comprising bicomponent fibers). Line parameters were a line speed of 290-305 m/min and a nip pressure of 56-66 N/mm. The bond pattern was an open dot bond pattern with a bonding area of 13.6%.

**Table 3**

| Ex. | PP S1 | coPP S1 | PP/coPP ratio S1 | BW S1 (g/m²) | PP S2 | coPP S2 | PP/coPP ratio S2 | BW S2 (g/m²) |
|---|---|---|---|---|---|---|---|---|
| 8 | 5,3 | 5,3 | 50/50 | 10,6 | 4,2 | 4,2 | 50/50 | 8,4 |
| 9 | 5,3 | 5,3 | 50/50 | 10,6 | 4,2 | 4,2 | 50/50 | 8,4 |
| 10 | 5,3 | 5,3 | 50/50 | 10,6 | 4,2 | 4,2 | 50/50 | 8,4 |
| 11 | 5,3 | 5,3 | 50/50 | 10,6 | 4,2 | 4,2 | 50/50 | 8,4 |

**Table 3 (cntd.)**

| Ex. | oTPE in MM | rem. MM | wt.% oTPE in MM | BW MM (g/m²) | Calender Temp. E-roll (°C) | Calender Temp. S-roll (°C) |
|---|---|---|---|---|---|---|
| 8 | | 708FB | 0 | 3,4 | 99 | 91 |
| 9 | | 708FB | 0 | 3,4 | 147 | 131 |
| 10 | EXP520 | | 100 | 3,4 | 147 | 131 |
| 11 | EXP520 | | 100 | 3,4 | 99 | 91 |

The 708FB material is a standard polypropylene homopolymer meltblown grade from the company Borealis with an MFR of 800 g/10min.

Examples 8, 9 and 10 are comparative examples outside the scope of the present invention, as they do not comprise a TPE material in the meltblown core layer (Examples 8, 9) and/or are not underbonded (Examples 9, 10). Example 11 is an inventive example. Measurement results for the nonwoven sheets of Examples 8-11 are shown in Table 4.

**Table 4**

| Ex. | CD tensile strength (TSCD) @break [N/50mm] | CD tensile elongation @break [%] | % force of TSCD at TE(100%) [%] |
|---|---|---|---|
| 8 | 11,3 | 106,3 | 95,2 |
| 9 | 16,8 | 174,4 | 72 |
| 10 | 23,7 | 238,5 | 42,6 |
| 11 | 19,3 | 265,8 | 34,9 |

All values from Table 4 were determined following the WSP 100.4 test method.

As apparent, the examples that use TPE in the meltblown layer (Examples 10 and 11) have better tensile strength and much better elongation than the examples using standard meltblown grade materials (examples 8 and 9). Also, as apparent from the last column of Table 4, both examples that use TPE in the meltblown layer (Examples 10 and 11) show that tensile @100% is much less than 0,65 and even less than 0,55 of tensile @break. This comparison between the CD tensile force at 100% stretch to the CD tensile force at break is representative for a very easy elongation in the most useful range of below 100% stretch, in particular between 50-100% stretch.

Within the examples that use TPE in the meltblown layer (Examples 10 and 11), the underbonded inventive examples (Example 11) shows still better properties of elongation @break (beyond 250%) and a quotient of tensile @100% to tensile @break of less than 0,40.

Within the examples that use standard meltblown grade materials (examples 8 and 9), underbonding as in Example 8 makes these properties much worse instead of better.

To summarize, when utilizing an elastic polymer in the meltblown layer, it is possible to underbond the spunbonded loft fibers (calender temperature below usual bonding temperatures). Then the spunbonded fibers will not be the limiting factor for the stretchability and the elastic polymer in the meltblown layer functions as an adhesive layer keeping the nonwoven web intact. The elastic polymer does not only facilitate higher stretchability itself, but also allows the spunbonded fibers to be notably underbonded, while keeping the nonwoven web from delaminating, which is normally seen when underbonding SS and SMS nonwoven (SMMS).

## Claims

1. A method for making a nonwoven sheet (100), the nonwoven sheet (100) comprising two facing layers (110, 120) of spunbonded nonwoven material and a middle layer (130) of meltblown nonwoven material, wherein the spunbonded material of the facing layers (110, 120) comprises crimped multicomponent fibers, the method comprising the following in-line steps:
spinning crimped multicomponent fibers and laying them onto a moving spinbelt to form a web preceding one of the facing layers (110);
meltblowing fibers and laying them onto the surface of the web formed in the previous step to form a web preceding the middle layer (130);
spinning crimped multicomponent fibers and laying them onto the surface of the web formed in the previous step to form a web preceding the other one of the facing layers (120); and
bonding the adjacent webs to form the nonwoven sheet (100) by point-bond hot calendering;
**characterized in that**
the fibers of the meltblown material of the middle layer (130) comprise a thermoplastic elastomer (TPE); and
that the temperature of the engraved roll (13) of the calender is at least 10°C and/or 5% lower than the melting temperature, as determined with DSC according to ISO 11357-3, of the lowest melting component of the multicomponent fibers.

2. The method of claim 1, wherein the temperature of the engraved roll (13) of the calender is least 20°C, preferably at least 30°C, more preferably at least 40°C lower than the melting temperature of the lowest melting component of the crimped multicomponent fibers, and/or wherein the temperature of the engraved roll (13) of the calender is least 10%, preferably at least 20%, more preferably at least 30% lower than the melting temperature of the lowest melting component of the crimped multicomponent fibers.

3. The method of any preceding claim, wherein the absolute temperature of the engraved roll (13) of the calender is 130°C or less, preferably 120°C or less, more preferably 110°C or less.

4. The method of any preceding claim, wherein the temperature of the smooth roll (14) of the calender is smaller than the temperature of the engraved roll (13) of the calender, wherein preferably the temperature difference between the engraved and smooth rolls (13, 14) of the calender is less than 20°C.

5. The method of any preceding claim, wherein the nip pressure of the calender is between 20 and 100 N/mm, preferably between 30 and 70 N/mm; and/or wherein the line speed of the nonwoven sheet (100) passing through the calender is between 100 and 400 n/min, preferably between 200 and 350 m/min.

6. The method of any preceding claim, wherein the engraved roll (13) is such that the number of bonding points per cm² of fabric surface is between 30 and 70, that the total area of the fabric surface taken up by the bonding points is between 8 and 15%, or both.

7. The method of any preceding claim, wherein one of the components of the crimped multicomponent fibers is a polypropylene (PP) material and another component of the crimped multicomponent fibers is either a different polypropylene (PP) material or a propylene-α-olefin copolymer (co-PP) material, preferably a poly(propylene-ethylene) copolymer material.

8. The method of claim 7, wherein the melting temperature of the polypropylene (PP) material, as determined with DSC according to ISO 11357-3, is between 150 and 170°C, preferably between 155 and 165°C; and/or wherein the melting temperature of the propylene-α-olefin copolymer (co-PP) material, as determined with DSC according to ISO 11357-3, is between 140 and 160°C, preferably between 145 and 155°C.

9. The method of any preceding claim, wherein the melt flow rates of all polymer materials used as components in the multicomponent fibers is between 10 and 50 g/10min, as determined according to ISO 1133 with conditions being 230°C and 2.16 kg.

10. The method of any preceding claim, wherein at least 10 wt.%, preferably at least 50 wt.%, more preferably at least 90 wt.% of the material forming for the meltblown fibers of the middle layer (130) is a thermoplastic elastomer (TPE) material; and/or wherein the thermoplastic elastomer (TPE) material is a thermoplastic polyolefin elastomer (TPE-o) material, preferably a thermoplastic polyolefin elastomer material comprising propylene-α-olefin copolymers.

11. A nonwoven sheet (100) made by a method of any preceding claim, the nonwoven sheet (100) comprising two facing layers (110, 120) of spunbonded nonwoven material and a middle layer (130) of meltblown nonwoven material, wherein the spunbonded material of the facing layers (110, 120) comprises crimped multicomponent fibers,
**characterized in that**
the meltblown material of the middle layer (130) comprises a thermoplastic elastomer;
the nonwoven sheet (100) is underbonded; and
the nonwoven sheet (100), in cross-machine direction (CD), has a tensile strength @100% elongation of less than 0,65 of its tensile strength @break, with tensile strength @100% elongation and tensile strength @break both being determined according to WSP 110.4.

12. The nonwoven sheet according to claim 11, the nonwoven sheet (100), in cross-machine direction (CD), has a tensile strength @100% elongation of less than 0,55 of its tensile strength @break, with tensile strength @100% elongation and tensile strength @break both being determined according to WSP 110.4.

13. The nonwoven sheet according to claim 11 or 12, wherein the nonwoven sheet (100) has an extension at break in cross-machine direction (CD), when measured according to WSP 110.4, of at least 150%, a CD elongation at 5N, when measured according to WSP 110.4, of at least 75%, or both.

14. A laminate comprising a nonwoven sheet (100) according to any one of claims 11 to 13 and an elastically stretchable material, preferably an elastically stretchable film.

15. A hygiene article comprising a nonwoven sheet (100) according to any one of claims 11 to 13 or a laminate according to claim 14, wherein preferably the hygiene article is an open diaper having diaper ears and the nonwoven sheet (100) or laminate forms at least part of the diaper ears.

## Patentansprüche

1. Verfahren zum Herstellen einer Vliesstofflage (100), wobei die Vliesstofflage (100) zwei Einfassungsschichten (110, 120) aus spinngebundenem Vliesstoffmaterial und eine mittlere Schicht (130) aus schmelzgeblasenem Vliesstoffmaterial umfasst, wobei das spinngebundene Material der Einfassungsschichten (110, 120) gekräuselte Mehrkomponentenfasern umfasst, wobei das Verfahren die folgenden integrierten Schritte umfasst:
Spinnen von gekräuselten Mehrkomponentenfasern und Auflegen dieser auf ein sich bewegendes Spinnband, um eine Bahn vor einer der Einfassungsschichten (110) auszubilden;
Schmelzblasen von Fasern und Auflegen dieser auf die Oberfläche der Bahn, die in dem vorhergehenden Schritt ausgebildet wurde, um eine Bahn vor der mittleren Schicht (130) auszubilden;
Spinnen von gekräuselten Mehrkomponentenfasern und Auflegen dieser auf die Oberfläche der Bahn, die in dem vorhergehenden Schritt ausgebildet wurde, um eine Bahn vor der anderen der Einfassungsschichten (120) auszubilden;
Binden der benachbarten Bahnen, um die Vliesstofflage (100) durch punktuelles Heißkalandrieren auszubilden;
**dadurch gekennzeichnet, dass**
die Fasern des schmelzgeblasenen Materials der mittleren Schicht (130) ein thermoplastisches Elastomer (TPE) umfassen; und
dass die Temperatur der gravierten Walze (13) des Kalanders mindestens 10 °C und/oder 5 % niedriger als die Schmelztemperatur, bestimmt mit DSC gemäß ISO 11357-3, der niedrigsten Schmelzkomponente der Mehrkomponentenfasern ist.

2. Verfahren nach Anspruch 1, wobei die Temperatur der gravierten Walze (13) des Kalanders (13) des Kalanders mindestens 20 °C, vorzugsweise mindestens 30 °C, noch bevorzugter mindestens 40 °C niedriger als die Schmelztemperatur der kleinsten Schmelzkomponente der gekräuselten Mehrkomponentenfasern ist, und/oder wobei die Temperatur der gravierten Walze (13) des Kalanders mindestens 10 %, vorzugsweise mindestens 20 %, noch bevorzugter mindestens 30 % niedriger als die Schmelztemperatur der niedrigsten Schmelzkomponente der gekräuselten Mehrkomponentenfasern ist.

3. Verfahren nach einem der vorhergehenden Ansprüche, wobei die absolute Temperatur der gravierten Walze (13) des Kalanders 130 °C oder weniger, vorzugsweise 120 °C oder weniger, noch bevorzugter 110 °C oder weniger beträgt.

4. Verfahren nach einem der vorhergehenden Ansprüche, wobei die Temperatur der glatten Walze (14) des Kalanders niedriger als die Temperatur der gravierten Walze (13) des Kalanders ist, wobei vorzugsweise der Temperaturunterschied zwischen der gravierten und der glatten Walze (13, 14) des Kalanders weniger als 20 °C beträgt.

5. Verfahren nach einem der vorhergehenden Ansprüche, wobei der Kalanderwalzendruck zwischen 20 und 100 N/mm, vorzugsweise zwischen 30 und 70 N/mm beträgt; und/oder wobei die Liniengeschwindigkeit der Vliesstofflage (100), die durch den Kalander läuft, zwischen 100 und 400 m/min, vorzugsweise zwischen 200 und 350 m/min beträgt.

6. Verfahren nach einem der vorhergehenden Ansprüche, wobei die gravierte Walze (13) so beschaffen ist, dass die Anzahl der Bindungspunkte pro cm² Gewebeoberfläche zwischen 30 und 70 liegt, dass die Gesamtfläche der Gewebeoberfläche, die durch die Bindungspunkte eingenommen wird, zwischen 8 und 15 % liegt oder beides.

7. Verfahren nach einem der vorhergehenden Ansprüche, wobei eine der Komponenten der gekräuselten Mehrkomponentenfasern ein Polypropylen-(PP-)Material ist und eine andere Komponente der gekräuselten Mehrkomponentenfasern entweder ein anderes Polypropylen-(PP-)Material oder ein Propylen-α-Olefin-Copolymer-(Co-PP-)Material, vorzugsweise ein Poly(propylen-ethylen)-Copolymer-Material ist.

8. Verfahren nach Anspruch 7, wobei die Schmelztemperatur des Polypropylen-(PP-)Materials, bestimmt mit DSC gemäß ISO 11357-3, zwischen 150 und 170 °C, vorzugsweise zwischen 155 und 165 °C liegt; und/oder wobei die Schmelztemperatur des Propylen-α-Olefin-Copolymer-(Co-PP-)Materials, bestimmt mit DSC gemäß ISO 11357-3, zwischen 140 und 160 °C, vorzugsweise zwischen 145 und 155 °C liegt.

9. Verfahren nach einem der vorhergehenden Ansprüche, wobei die Schmelzflussraten aller Polymermaterialien, die als Komponenten in den Mehrkomponentenfasern verwendet werden, zwischen 10 und 50 g/10 min liegen, bestimmt gemäß ISO 1133 mit Bedingungen von 230 °C und 2,16 kg.

10. Verfahren nach einem der vorhergehenden Ansprüche, wobei mindestens 10 Gew.-%, vorzugsweise mindestens 50 Gew.-%, noch bevorzugter mindestens 90 Gew.-% des Materials, das die schmelzgeblasenen Fasern der mittleren Schicht (130) ausbildet, ein thermoplastisches Elastomer-(TPE-)Material ist; und/oder wobei das thermoplastische Elastomer-(TPE-)Material ein thermoplastisches Polyolefinelastomer-(TPE-o-)Material ist, vorzugsweise ein thermoplastisches Polyolefinelastomermaterial, umfassend Propylen-α-Olefin-Copolymere.

11. Vliesstofflage (100), hergestellt durch ein Verfahren nach einem der vorhergehenden Ansprüche, wobei die Vliesstofflage (100) zwei Einfassungsschichten (110, 120) aus spinngebundenem Vliesstoffmaterial und eine mittlere Schicht (130) aus schmelzgeblasenem Vliesstoffmaterial umfasst, wobei das spinngebundene Material der Einfassungsschichten (110, 120) gekräuselte Mehrkomponentenfasern umfasst,
**dadurch gekennzeichnet, dass**
das schmelzgeblasene Material der mittleren Schicht (130) ein thermoplastisches Elastomer umfasst;
die Vliesstofflage (100) untergebunden ist; und
die Vliesstofflage (100) in Maschinenquerrichtung (CD) eine Zugfestigkeit bei 100 % Dehnung von weniger als 0,65 ihrer Zugfestigkeit am Bruch aufweist, wobei die Zugfestigkeit bei 100 % Dehnung und die Zugfestigkeit am Bruch beide gemäß WSP 110.4 bestimmt werden.

12. Vliesstofflage nach Anspruch 11, wobei die Vliesstofflage (100) in Maschinenquerrichtung (CD) eine Zugfestigkeit bei 100 % Dehnung von weniger als 0,55 ihrer Zugfestigkeit am Bruch aufweist, wobei die Zugfestigkeit bei 100 % Dehnung und die Zugfestigkeit am Bruch beide gemäß WSP 110.4 bestimmt werden.

13. Vliesstofflage nach Anspruch 11 oder 12, wobei die Vliesstofflage (100) eine Bruchdehnung in Maschinenquerrichtung (CD), gemessen gemäß WSP 110.4, von mindestens 150 %, eine CD-Dehnung bei 5N, gemessen gemäß WSP 110.4, von mindestens 75 % oder beides aufweist.

14. Laminat, umfassend eine Vliesstofflage (100) nach einem der Ansprüche 11 bis 13 und ein elastisch dehnbares Material, vorzugsweise eine elastisch dehnbare Folie.

15. Hygieneartikel, umfassend eine Vliesstofflage (100) nach einem der Ansprüche 11 bis 13 oder ein Laminat nach Anspruch 14, wobei der Hygieneartikel vorzugsweise eine offene Windel mit Windelohren ist und die Vliesstofflage (100) oder das Laminat mindestens einen Teil der Windelohren ausbildet.

## Revendications

1. Procédé de fabrication d'une feuille non tissée (100), la feuille non tissée (100) comprenant deux couches opposées (110, 120) d'un matériau non tissé filé-lié et une couche centrale (130) d'un matériau non tissé obtenu par fusion-soufflage, dans lequel le matériau filé-lié des couches opposées (110, 120) comprend des fibres frisées à composants multiples, dans lequel le procédé comprend les étapes en ligne suivantes :
le filage de fibres frisées à composants multiples et leur placement sur une sangle de filature mobile pour former une toile précédant une des couches opposées (110) ;
le soufflage-fusion de fibres et leur placement sur la surface de la toile formée lors de l'étape précédente pour former une toile précédant la couche centrale (130) ;
le filage de fibres frisées à composants multiples et leur placement sur la surface de la toile formée lors de l'étape précédente pour former une toile précédant l'autre des couches opposées (120) ; et
la liaison des tissus adjacents pour former la feuille non tissée (100) par calandrage à chaud de liaison de points,
**caractérisé en ce que**
les fibres du matériau obtenues par fusion-soufflage de la couche centrale (130) comprennent un élastomère thermoplastique (TPE) ; et
que la température du cylindre gravé (13) de la calandre est d'au moins 10 °C et/ou est inférieure de 5 % à la température de fusion, telle que déterminée avec DSC selon la norme ISO 11357-3 du composant au point de fusion le plus bas des fibres à composants multiples.

2. Procédé selon la revendication 1, dans lequel la température du cylindre gravé (13) de la calandre est d'au moins 20 °C, de préférence d'au moins 30 °C, de manière davantage préférée d'au moins 40 °C plus basse que la température de fusion du composant au point de fusion le plus bas des fibres frisées à composants multiples et/ou dans lequel la température du cylindre gravé (13) de la calandre est d'au moins 10 %, de préférence d'au moins 20 %, de manière davantage préférée d'au moins 30 % inférieure à la température de fusion du composant au point de fusion le plus bas des fibres frisées à composants multiples.

3. Procédé selon l'une quelconque des revendications précédentes, dans lequel la température absolue du cylindre gravé (13) de la calandre est de 130 °C ou moins, de manière préférée de 120 °C ou moins, de manière davantage préférée de 110 °C ou moins.

4. Procédé selon l'une quelconque des revendications précédentes, dans lequel la température du cylindre lisse (14) de la calandre est inférieure à la température du cylindre gravé (13) de la calandre, dans lequel de manière préférée la différence de température entre les cylindres gravé et lisse (13, 14) de la calandre est inférieure à 20 °C.

5. Procédé selon l'une quelconque des revendications précédentes, dans lequel la pression de pincement de la calandre est comprise entre 20 et 100 N/mm, de manière préférée entre 30 et 70 N/mm ; et/ou dans lequel la vitesse linéaire de la feuille non tissée (100) passant par la calandre est comprise entre 100 et 400 n/min, de manière préférée entre 200 et 350 m/min.

6. Procédé selon l'une quelconque des revendications précédentes, dans lequel le cylindre gravé (13) est tel que le nombre de points de liaison par cm² de la surface de tissu est compris entre 30 et 70, que la superficie totale de la surface de tissu reprise par les points de liaison est comprise entre 8 et 15 % ou les deux.

7. Procédé selon l'une quelconque des revendications précédentes, dans lequel un des composants des fibres frisées à composants multiples est un matériau en polypropylène (PP) et un autre composant des fibres frisées à composants multiples est soit un matériau en polypropylène (PP) différent soit un matériau en copolymère propylène-α-oléfine (co-PP), de manière préférée un matériau en copolymère de poly(propylène-éthylène).

8. Procédé selon la revendication 7, dans lequel la température de fusion du matériau en polypropylène (PP), telle que déterminée avec DSC selon la norme ISO 11357-3, est comprise entre 150 et 170 °C, de manière préférée entre 155 et 165 °C ; et/ou dans lequel la température de fusion du matériau en copolymère de propylène-α-oléfine (co-PP), telle que déterminée avec DSC selon la norme ISO 11357-3, est comprise entre 140 et 160 °C, de manière préférée entre 145 et 155 °C.

9. Procédé selon l'une quelconque des revendications précédentes, dans lequel l'indice de fluage à l'état fondu de tous les matériaux polymères utilisés en tant que composants dans les fibres à composants multiples est compris entre 10 et 50 g/min, tel que déterminé selon la norme ISO 133 dans des conditions de 230 °C et 2,16 kg.

10. Procédé selon l'une quelconque des revendications précédentes, dans lequel au moins 10 % en poids, de manière préférée au moins 50 % en poids, de manière davantage préférée au moins 90 % en poids de la formation de matériau pour les fibres obtenues par fusion-soufflage de la couche centrale (130) est un matériau en élastomère thermoplastique (TPE) ; et/ou dans lequel le matériau en élastomère thermoplastique (TPE) est un matériau en élastomère de polyoléfine thermoplastique (TPE-o), de manière préférée un matériau en élastomère de polyoléfine thermoplastique comprenant des copolymères de propylène-α-oléfine.

11. Feuille non tissée (100) fabriquée par un procédé selon l'une quelconque des revendications précédentes, la feuille non tissée (100) comprenant deux couches opposées (110, 120) d'un matériau non tissé filé-lié et une couche centrale (130) d'un matériau non tissé obtenu par fusion-soufflage, dans laquelle le matériau filé-lié des couches opposées (110, 120) comprend des fibres frisées à composants multiples,
**caractérisée en ce que**
le matériau obtenu par fusion-soufflage de la couche centrale (130) comprend un élastomère thermoplastique ;
la feuille non tissée (100) est sous-liée ; et
la feuille non tissée (100), dans une direction transversale de machine (CD), présente une résistance à l'allongement par traction à 100 % inférieure à 0,65 de sa résistance à la rupture par traction, la résistance à l'allongement par traction à 100 % et la résistance à la rupture par traction étant déterminées toutes deux selon la norme WSP 110.4.

12. Feuille non tissée selon la revendication 11, dans laquelle la feuille non tissée (100), dans la direction transversale de machine (CD), présente une résistance à l'allongement par traction à 100 % inférieure à 0,55 de sa résistance à la rupture par traction, la résistance à l'allongement par traction à 100 % et la résistance à la rupture par traction étant toutes deux déterminées selon la norme WSP 110.4.

13. Feuille non tissée selon la revendication 11 ou 12, dans laquelle la feuille non tissée (100) présente un allongement à la rupture dans la direction de machine transversale (CD) lorsqu'elle est mesurée selon la norme WSP 110.4, d'au moins 150 %, un allongement CD à 5 N lorsqu'il est mesuré selon la norme WSP 110.4 d'au moins 75 %, ou les deux.

14. Stratifié comprenant une feuille non tissée (100) selon l'une quelconque des revendications 11 à 13 et un matériau élastiquement étirable, de manière préférée un film élastiquement étirable.

15. Article d'hygiène comprenant une feuille non tissée (100) selon l'une quelconque des revendications 11 à 13 ou stratifié selon la revendication 14, dans lequel de manière préférée l'article d'hygiène est une couche ouverte présentant des oreilles de couche et la feuille non tissée (100) ou le stratifié forme au moins une partie des oreilles de couche.
